# EUROPEAN PATENT SPECIFICATION

(11) **EP 2 435 002 B1**
(45) Date of publication and mention of the grant of the patent: **09.10.2013**
(21) Application number: 10714100.4
(22) Date of filing: 09.03.2010
(51) Int. Cl.: A61F 13/15

(54) **PANTS-TYPE DISPOSABLE DIAPER**
EINWEGWINDEL VOM HÖSCHENTYP
COUCHE JETABLE DE TYPE CULOTTE

(30) Priority: 29.05.2009 JP 2009130888
(43) Date of publication of application: 04.04.2012
(73) Proprietor: Livedo Corporation, Shikokuchuo-shi Ehime 799-0122 (JP)
(72) Inventor: TAKAHASHI, Yuki, Mima-gun Tokushima 779-4104 (JP); NAKAOKA, Kenji, Osaka-shi Osaka 541-0048 (JP)
(74) Representative: Gulde Hengelhaupt Ziebig & Schneider
(86) International application number: PCT/JP2010/054284
(87) International publication number: WO 2010/137383

(56) References cited:
- WO-A1-98/20822
- WO-A1-2010/017158
- JP-A- 2007 097 979
- US-A1- 2003 106 560
- US-A1- 2004 091 677
- US-A1- 2006 135 923

## Description

### TECHNICAL FIELD

The present invention relates to a pants-type disposable diaper for an infant or an adult.

### BACKGROUND ART

Conventinally, there is known a pants-type disposable diaper comprising: a pants-shaped outer member including an inner sheet and an outer sheet; and an absorbent main body disposed on an inner surface of the pants-shaped outer member. Japanese Laid-Open Patent Publication No. 2007-097979 (Patent Literature 1) discloses that a hydrophilized sheet that is prepared by immersing a spunbonded nonwoven fabric formed from a polypropylene or a polyolefin/polyester copolymer in a surfactant, is used in the pants-type disposable diaper as the inner sheet and the outer sheet of the pants-shaped outer member. In addition, Patent Literature 1 discloses that when the spunbonded nonwoven fabric, that is formed from the polypropylene or the polyolefin/polyester copolymer, is immersed in the surfactant, a surface of the nonwoven fabric sheet is smoothed and a skin feel of the nonwoven fabric sheet is improved.

### CITATION LIST

### PATENT LITERATURE

PATENT LITERATURE 1
   Japanese Laid-Open Patent Publication No. 2007-097979

### SUMMARY OF INVENTION

### TECHNICAL PROBLEM

In the pants-type disposable diaper disclosed in Patent Literature 1, a skin feel of the nonwoven fabric, which is used as the inner sheet and the outer sheet of the pants-shaped outer member, is improved by immersing the nonwoven fabric in the surfactant. However, since the nonwoven fabric is hydrophilized at the same time, water resistance of the pants-shaped outer member decreases. Therefore, there is room for improvements in leakage prevention.

WO 2010/017158 A1 which is prior art according to Article 54 (3) discloses an absorbent article that comprises a barrier component of a nonwoven barrier sheet usable for a back sheet, comprising a spunbond nonwoven web and a meltblown nonwoven web. The document further describes that the surface of the barrier component may be coated with a skin care composition, and the skin care composition may comprise a solid nonionic surfactant. Thus, this nonionic surfactant is not melted with a polymer to form a nonwoven web, but is a component of the skin care composition. US 2004/091677 A1 discloses a multi-microlayer thermoplastic film having a plurality of corrugated polymer layers and a plurality of thermoplastic elastomer layers which may comprise filler materials and surfactants. US 2003/0106560 A1 describes a single-use, disposable absorbent laminate containing one or more layers of hydrophilic meltspun material bonded to a breathable film. The meltspun fabric layer may include at least one spunbonded fabric that is made hydrophilic prior to bonding to the film. The filaments or microfibers of the spunbonded or meltblown fabrics may contain a hydrophilic additive in or on the filaments or microfibers. US 2006/0135923 A1 discloses a nonwoven fabric composite that comprises at least one extensible layer of elastomeric fine fibers treated with a wetting agent. The nonwoven fabric composite can be used as barrier layer or a wrap for an absorbent materials to prevent particles form migrating out of the absorbent material.

WO 98/20822 A1 describes a disposable absorbent article such as an absorbent diaper comprising a topsheet, a backsheet and an absorbent core disposed between the topsheet and the backsheet. The backsheet comprises nonwoven web. The absorbent article has a soft-clothlike backsheet which comprises a plastic film having an outer-facing surface and a body-facing surface, and the nonwoven web is joined with the outer-facing surface of the plastic film to form a laminate. The disclosed absorbent article does not have an outer member of pants shape.

The present invention has been achieved in view of the above circumstances, and an object of the present invention is to provide a pants-type disposable diaper comprising a pants-shaped outer member that is excellent in water resistance and softness.

### SOLUTION TO PROBLEM

A pant-type disposable diaper of the present invention which solves the above problems comprises: a pants-shaped outer member having a front part, a back part, and a crotch part positioned between the front part and the back part, and having a waist opening and a pair of leg openings formed by joining the front part and the back part; and an absorbent main body disposed on an inner surface of the pants-shaped outer member at the crotch part, and comprising a top sheet, a back sheet, and an absorbent core disposed between the top sheet and the back sheet; wherein the pants-shaped outer member comprises an inner sheet and an outer sheet, and the inner sheet and the outer sheet are made of a spunbonded nonwoven fabric produced by melting a polymer composition containing polyolefin and/or polyester and a nonionic surfactant.

In the present invention, a nonwoven fabric used for the inner sheet and an outer sheet of the pants-shaped outer member is not obtained by immersing the nonwoven fabric in a surfactant, but is obtained by forming the nonwoven fabric from the polymer composition, a raw material, in which a nonionic surfactant is contained. Therefore, hydrophobicity provided by polyolefin and/or polyester is maintained in the obtained nonwoven fabric, and hence the nonwoven fabric is excellent in water resistance, and further softness is imparted to the nonwoven fabric. As a result, a skin feel of the diaper against a wearer is improved and a hand feel of the diaper is improved when a caregiver or the like handles the diaper, thereby providing a comfortable feeling.

The surfactant contained in the polymer composition is a nonionic surfactant. When a nonionic surfactant is used as the surfactant, hydrophobicity of the polyolefin and/or polyester nonwoven fabric tends to be maintained, and softness is easily imparted to the nonwoven fabric.

The nonwoven fabrics of the inner sheet and the outer sheet preferably have a fineness of 1.0 dtex or more and less than 1.5 dtex. When the nonwoven fabrics have a fineness of less than 1.5 dtex, softness is easily imparted to the nonwoven fabric. When the nonwoven fabrics have a fineness of 1.0 dtex or more, the spunbonded nonwoven fabric is easily produced.

The outer sheet is preferably folded back toward the inner sheet at an edge of the waist opening of the pants-shaped outer member. When the outer sheet is folded back at the edge of the waist opening toward the inner sheet side, a certain amount of thickness is provided to a circumference of the waist opening of the pants-shaped outer member. Therefore, even when the pants-shaped outer member is soft, handleability of the circumference of the waist opening of the pants-shaped outer member is improved, and it is easy to wear or remove the diaper

Preferably, a plurality of body elastic members are disposed between the inner sheet and the outer sheet at the front part and the back part of the pants-shaped outer member so as to extend in a width direction of the diaper, the body elastic members are adhered to the inner sheet and/or the outer sheet, and the inner sheet and the outer sheet are not adhered each other at a position between the adjacent body elastic members. The body elastic member improves a fitting property of the diaper around abdomen and hip regions. In addition, when the inner sheet and the outer sheet are not adhered each other at the position between the adjacent body elastic members, a hand feel of the diaper is further improved around a wearer's trunk.

### ADVANTAGEOUS EFFECTS OF INVENTION

The pants-type disposable diaper of the present invention comprises the pants-shaped outer member that is excellent in water resistance and softness, and hence, leakage of urine and the like is easily prevented, and a hand feel in handling the diaper and a skin feel of the diaper against the wearer are improved.

### BRIEF DESCRIPTION OF DRAWINGS

Fig. 1 shows a perspective view of a pants-type disposable diaper of the present invention.
Fig. 2 shows a plan view of the pants-type disposable diaper shown in Fig. 1 in a developed state in which a front part and a back part are disjoined.
Fig. 3 shows a cross-sectional view taken along line III-III in Fig. 2.
Fig. 4 shows a cross-sectional view taken along line IV-IV in Fig. 2.
Fig. 5 shows a cross-sectional view taken along line V-V in Fig. 2.
Figs. 6A, 6B and 6C show examples of applying a hot-melt adhesive to a body elastic member.

### DESCRIPTION OF EMBODIMENTS

A pants-type disposable diaper of the present invention comprises: a pants-shaped outer member having a front part, a back part, and a crotch part positioned between the front part and the back part, and having a waist opening and a pair of leg openings formed by joining the front part and the back part; and an absorbent main body disposed on an inner surface of the pants-shaped outer member at the crotch part, and comprising a top sheet, a back sheet, and an absorbent core disposed between the top sheet and the back sheet.

In the pants-shaped outer member, the front part and the back part are joined at their both side edges in a width direction of the diaper, whereby forming the pair of leg openings on both sides of the crotch part and the waist opening provided by edges of end parts, with respect to a front-back direction of the diaper, of the front part and the back part. Here, the front-back direction means a direction from the front part toward the back part of the pants-type disposable diaper. The width direction means a direction orthogonal to the front-back direction on the same plane as the pants-type disposable diaper in a state where the front part and the back part of the pants-type disposable diaper are disjoined and the pants-type disposable diaper is developed on a plane.

Concerning names of respective parts of the pants-shaped outer member, a part applied to an abdomen side of a wearer is called the front part, a part applied to a buttocks side of the wearer is called the back part, and a part positioned between the front part and the back part and applied to a crotch of the wearer is called the crotch part, in a state of wearing the pants-type disposable diaper. The crotch part is a middle part when the pants-type disposable diaper is divided into three parts in the front-back direction in a state where the front part and the back part of the diaper are disjoined and the diaper is developed on a plane, and the crotch part is a part whose side edges in the width direction are not joined when the diaper is formed in a shape of pants.

The absorbent main body is disposed on the inner surface of the pants-shaped outer member at the crotch part. The inner surface means a surface that faces a wearer's skin in wearing the pants-type disposable diaper. An outer surface means a surface that faces outside in wearing the pants-type disposable diaper. The absorbent main body is disposed at least at the crotch part, and further, may be also extended to the front part and/or the back part. The absorbent main body absorbs urine and the like excreted from a wearer.

The pants-shaped outer member comprises a sheet member made of a spunbonded nonwoven fabric produced by melting a polymer composition containing polyolefin and/or polyester and a surfactant. This will be described below in detail.

The sheet member constituting the pants-shaped outer member is preferably made of a liquid-impermeable or water-repellent nonwoven fabric in light of leakage prevention. Thus, a nonwoven fabric that is formed from a hydrophobic fiber is preferable as the nonwoven fabric. In light of degree of hydrophobicity and ease of production, a polyolefin and/or polyester fiber is preferable as the hydrophobic fiber. However, a polyolefin and/or polyester nonwoven fabric is inferior softness, and may be felt stiff when handled. For that reason, in the present invention, when a nonwoven fabric used for the sheet member is produced, a nonionic surfactant is used in addition to polyolefin and/or polyester as a raw material of the nonwoven fabric. Thus, a nonwoven fabric produced by melting a polymer composition containing polyolefin and/or polyester and a nonionic surfactant is used as the sheet member constituting the pants-shaped outer member. As a result, softness is imparted to the sheet member.

In the present invention, a nonionic surfactant is used as one component of the polymer composition, and the nonwoven fabric is produced by melting the polymer composition, whereby softness is imparted to the nonwoven fabric without impairing hydrophobicity of the polyolefin and/or polyester nonwoven fabric at a marked degree. For example, when a polyolefin and/or polyester nonwoven fabric is used as a nonwoven fabric and the nonwoven fabric is immersed in a surfactant or a surfactant is applied to a surface of the nonwoven fabric, softness can be imparted to the nonwoven fabric but the polyolefin and/or polyester nonwoven fabric is hydrophilized. Thus, it is difficult to obtain a liquid-impermeable or water-repellent nonwoven fabric. However, in the present invention, since a nonionic surfactant is blended in a raw material of a fiber for forming a nonwoven fabric to obtain the polymer composition, hydrophobicity provided by the polyolefin and/or polyester is maintained in the obtained nonwoven fabric, and hence the nonwoven fabric becomes excellent in water resistance. Therefore, a liquid-impermeable or water-repellent nonwoven fabric with softness is obtained.

Further, in the present invention, a surfactant is used as one component of the polymer composition, and the nonwoven fabric is produced by melting the polymer composition, thereby obtaining a nonwoven fabric having an excellent adherence property. For example, when a nonwoven fabric is immersed in a surfactant or a surfactant is applied to a surface of the nonwoven fabric, it may be difficult to adhere an elastic member to the obtained nonwoven fabric with an adhesive, it may be difficult to adhere the obtained nonwoven fabrics to each other with an adhesive, or the adhesive strength may decrease. However, in the present invention, since a nonionic surfactant is blended in a raw material of a fiber for forming a nonwoven fabric to obtain the polymer composition, the adherence property of the obtained nonwoven fabric can be almost prevented from deteriorating.

As the polyolefin contained in the polymer composition, a polyolefin that is commonly used for a nonwoven fabric can be used. For example, polyethylene, polypropylene, polybutene-1, polyisobutylene or the like may be used. Among them, polyethylene and/or polypropylene are preferable in light of ease and cost of production. These polyolefins may be used either alone or as a combination of at least two of them.

As the polyester contained in the polymer composition, a polyester that is commonly used for a nonwoven fabric can be used. For example, polyethylene terephthalate (PET), polybutylene terephthalate (PBT), polytrimethylene terephthalate (PTT) or the like may be used. These polyesters may be used either alone or as a combination of at least two of them.

As the surfactant, a nonionic surfactant is used. Since a nonionic surfactant has a hydrophilic part of nonionic, using a nonionic surfactant as one component of the polymer composition enables easily to maintain hydrophobicity of the polyolefin and/or polyester nonwoven fabric. In addition, a nonionic surfactant has a relatively excellent affinity with polyolefin and polyester, and hence softness is easily imparted to the nonwoven fabric.

Examples of the nonionic surfactants include polyalcohol fatty acid esters such as sucrose fatty acid esters, glycerin fatty acid esters, and sorbitan fatty acid esters; polyoxyethylene alkyl ethers; polyoxyethylene alkyl phenyl ether; polyoxyethylene fatty acid esters; and polyoxyethylene polyalcohol fatty acid esters. These nonionic surfactants may be used either alone or as a combination of at least two of them.

A content of the surfactant in the polymer composition is preferably 0.5 mass % or more, more preferably 1.0 mass % or more, preferably 5.0 mass % or less, and more preferably 3.5 mass % or less. When the content of the surfactant in the polymer composition is in the range of from 0.5 mass% to 5.0 mass %, softness and hydrophobicity of the nonwoven fabric which is prepared by melting the polymer composition are ensured sufficiently.

As the nonwoven fabric constituting the sheet member, a spunbonded nonwoven fabric is preferable. Here, the spunbonded nonwoven fabric means a nonwoven fabric manufactured by a spunbonding method. The spunbonded nonwoven fabric is obtained, for example, by: melting the polymer composition; extruding the polymer composition from a spinning nozzle to be extended; collecting the extended polymer composition on a conveyor belt or the like to be formed into a web shape. In the present invention, since the spunbonded nonwoven fabric is used as the sheet member, breathability is ensured and imperviousness of moisture during wearing the diaper is easily prevented.

In the present invention, the spunbonded nonwoven fabric means a nonwoven fabric consisting of a spunbond layer. For example, an SMS nonwoven fabric in which a meltblown layer is interposed between spunbond layers is not preferable, because the meltblown layer impairs breathability. In addition, as described above, in the present invention, since the sheet member has a sufficient water resistance, it is unnecessary to provide a meltblown layer for enhancing water resistance of the sheet member.

The nonwoven fabric used for the sheet member has preferably a mass per unit area of 10 g/m² or more, more preferably 15 g/m² or more, preferably 35 g/m² or less, and more preferably 25 g/m² or less. When the nonwoven fabric has a mass per unit area of 10 g/m² or more, the sheet member tends to have a sufficient strength. When the nonwoven fabric has a mass per unit area of 35 g/m² or less, breathability of the nonwoven fabric is easily ensured, resulting in improving a comfort of a wearer.

The nonwoven fabric used for the sheet member has preferably a fineness of 1.0 dtex or more and less than 1.5 dtex. When the nonwoven fabric has a fineness of less than 1.5 dtex, softness is easily imparted to the nonwoven fabric. When the nonwoven fabric has a fineness of 1.0 dtex or more, the spunbonded nonwoven fabric is easily produced.

In the pants-type disposable diaper of the present invention, the pants-shaped outer member may comprise either only one or more of the sheet members. Preferably, the pants-shaped outer member comprise an inner sheet and an outer sheet, and the sheet member is used as the inner sheet and the outer sheet. That is, the inner sheet and the outer sheet are made of a spunbonded nonwoven fabric produced by melting a polymer composition containing polyolefin and/or polyester and a nonionic surfactant. The inner sheet is lamineted on an inner surface of the outer sheet. When the pants-shaped outer member comprises the inner sheet and the outer sheet, and the sheet member is used as the inner sheet and the outer sheet, a shape of the pants-shaped outer member is easily maintained to be a pants shape, and the pants-shaped outer member comes to be excellent in water resistance and breathability and further excellent in softness. As a result, a skin feel of the diaper against a wearer is improved and a hand feel of the diaper is improved when a caregiver or the like handles the diaper, thereby providing a comfortable feeling.

When the pants-shaped outer member comprises the inner sheet and the outer sheet, the outer sheet is preferably folded back toward the inner sheet at a waist opening edge of the pants-shaped outer member. Here, the waist opening edge means an edge of the waist opening in the pants-shaped outer member. When the outer sheet is folded back at the waist opening edge toward the inner sheet, a certain amount of thickness is provided to a circumference of the waist opening of the pants-shaped outer member. Therefore, even when the pants-shaped outer member is soft, handleability of the circumference of the waist opening of the pants-shaped outer member is improved, and it is easy to wear or remove the diaper. In addition, because the outer sheet is folded back toward the inner sheet, the appearance of the pants-shaped outer member is enhanced.

An elastic member is preferably disposed between the inner sheet and the outer sheet In this case, the elastic member may be adhered to the inner sheet and/or the outer sheet.

A waist elastic member is preferably disposed to the pants-shaped outer member along the waist opening edge at the front part and the back part. The waist elastic member prevents excrement such as urine and the like from leaking from a back side or an abdomen side, even when a wearer lies. The waist elastic member may be composed of a plurality of elastic members.

A leg elastic member is preferably disposed to the pants-shaped outer member along a leg opening edge. The leg elastic member prevents excrement such as urine and the like from leaking from the leg opening edge. Here, the leg opening edge means an edge of the leg opening in the pants-shaped outer member. The leg elastic member may be composed of a plurality of elastic members.

A plurality of body elastic members are preferably disposed to the pants-shaped outer member at the front part and the back part so as to extend in the width direction of the diaper. The body elastic member improves a fitting property of the diaper around abdomen and hip regions.

When the body elastic member is disposed to the pants-shaped outer member, the inner sheet and the outer sheet are not adhered each other at a position between the adjacent body elastic members, preferably. When the inner sheet and the outer sheet are not adhered each other at the position between the adjacent body elastic members, a hand feel of the diaper is further improved around a wearer's trunk.

Elastic materials such as a polyurethane thread, a polyurethane film, a natural rubber and the like, which are generally used for disposable diapers, can be used for the respective elastic members. The respective elastic members are preferably fixed to the inner sheet and/or the outer sheet in a stretched state with a hot-melt adhesive. For example, a polyurethane thread having a fineness of 100 dtex to 2,500 dtex is stretched at a ratio of 1.1 to 5.0 times to be fixed. A preferable bonding means is a rubber hot-melt adhesive.

In order not to adhere the inner sheet and the outer sheet at the position between the adjacent body elastic members, the following method may be employed. As an adhesive applicator, an adhesive application nozzle in which a pair of guides extends from a nozzle discharge opening is used, and the adhesive application nozzles, the number of which is equal to the number of elastic members, are arranged. While the adhesive is discharged from the nozzle discharge opening, each elastic member is reeled out through the paired guides to apply the adhesive around the elastic member. The plurality of elastic members to which the adhesive has been applied are disposed on the outer sheet. Then, the inner sheet is laminated on the outer sheet to which the elastic members have been fixed, and the inner sheet and the outer sheet are joined to each other by means of an adhesive, heat-sealing, or the like. It is noted that the above-described adhesive application nozzle is disclosed in Japanese Laid-Open Patent Publication No. 2009-11890.

The absorbent main body disposed on the inner surface of the pants-shaped outer member at the crotch part comprises a top sheet, a back sheet, and an absorbent core disposed between the top sheet and the back sheet. The top sheet is preferably made of a liquid-permeable nonwoven fabric or the like, and the back sheet is preferably made of a nonwoven fabric, a plastic film or the like which is liquid-impermeable or water-repellent.

The absorbent core is not particularly limited as long as it absorbs excrement such as urine and the like; however, it preferably contains an absorbent resin. The absorbent core can be obtained, for example, by the steps of mixing a hydrophilic fiber assembly such as crushed pulp fibers, cellulose fibers and the like with a granular absorbent resin to obtain a clump; wrapping the clump with a paper sheet such as a tissue paper and the luke, or with a cover sheet such as a liquid-permeable nonwoven fabric sheet and the like; and molding the obtained wrapped clump into a predefined shape such as a rectangular shape, an hourglass shape, a center nipped-in gourd shape, a battledore shape, and the like,

Rising flaps are preferably provided along edges of opposite sides, with respect to the width direction, of the absorbent main body. For example, the rising flaps may be joined to the top sheet of the absorbent main body, or may be provided outside the absorbent main body in the width direction. The rising flaps are preferably made of a nonwoven fabric, a plastic film or the like which is liquid-impermeable or water-repellent, and more preferably made of a water-repellent nonwoven fabric. Providing the rising flaps enables to prevent lateral leakage of urine and the like.

A rising elastic member is preferably disposed at an upper end (an end nearer to a wearer) of the rising flap being in a rising state. The rising flap forms a rising gather which rises toward a wearer due to a shrinkage force of the rising elastic member, thereby preventing lateral leakage of urine and the like. An inner surface of the rising flap may be joined to the top sheet at ends, with respect to the front-back direction of the diaper, of the rising flaps, thereby preventing leakage of urine and the like in the front-back direction.

Next, an example of the pants-type disposable diaper of the present invention is explained, referring to drawings. However, the present invention is not restricted to the following embodiment.

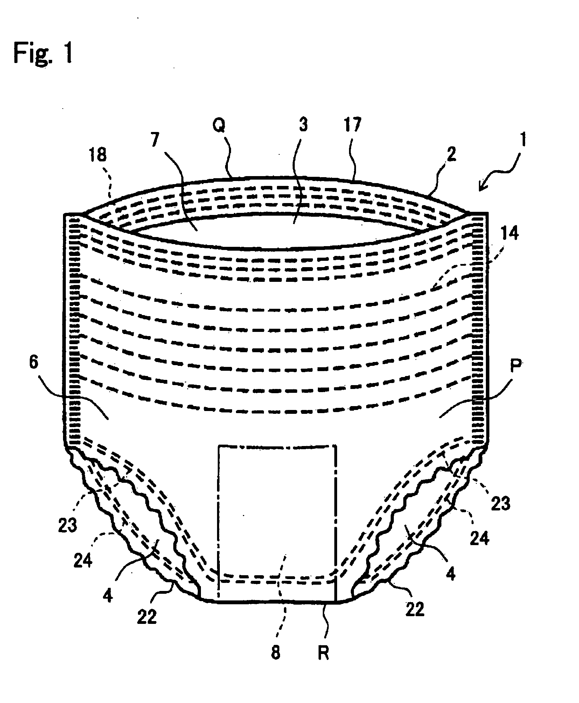

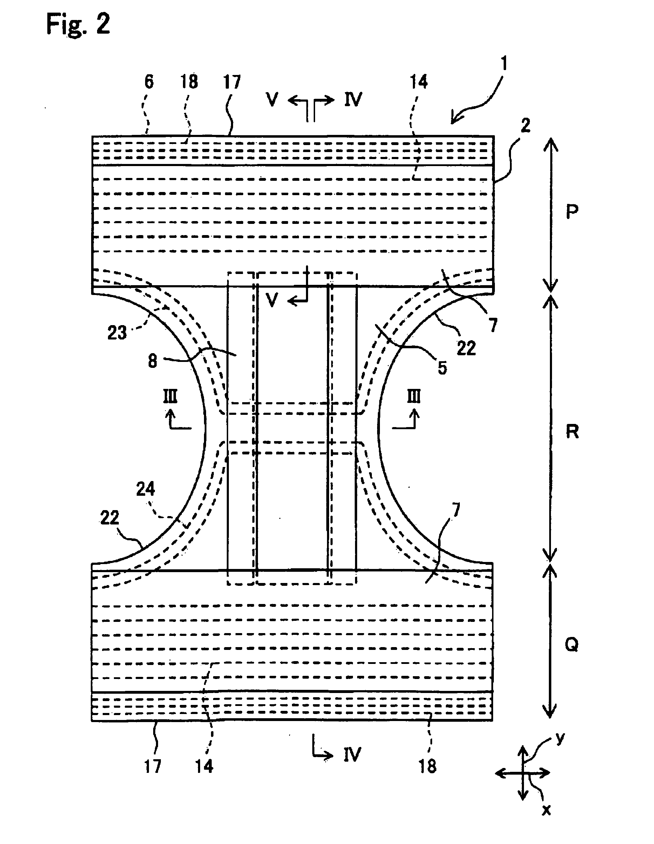

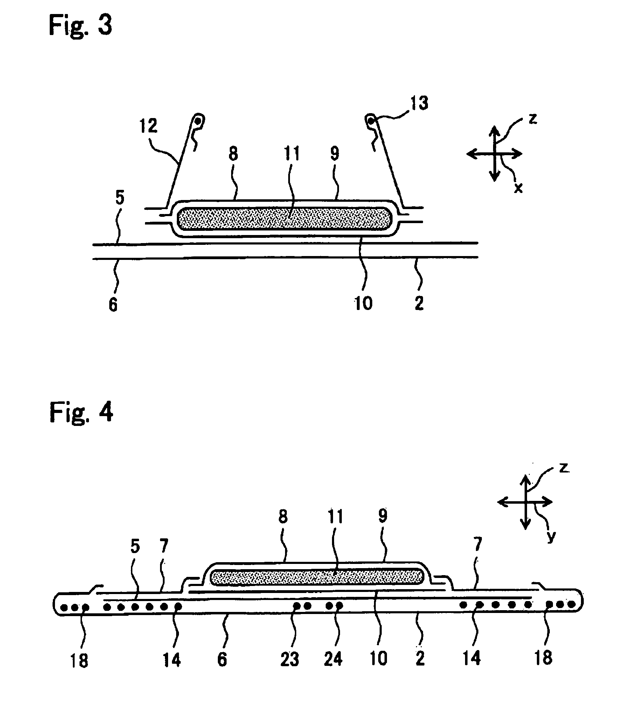

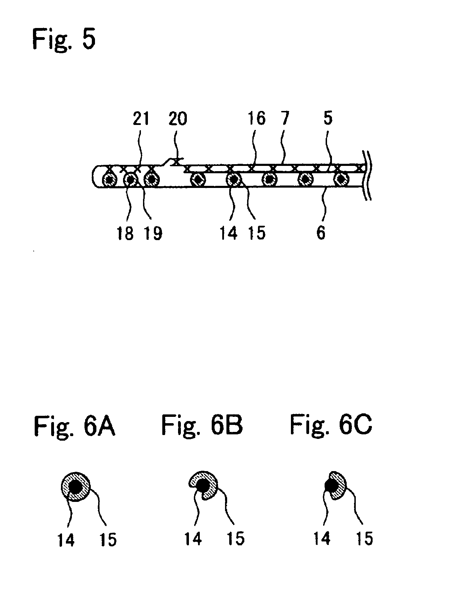

Fig. 1 shows a perspective view of a pants-type disposable diaper of the present invention. Fig. 2 shows a plan view of the pants-type disposable diaper shown in Fig. 1 in a developed state in which a front part and a back part are disjoined. Fig. 3 shows a cross-sectional view taken along line III-III in Fig. 2. Fig. 4 shows a cross-sectional view taken along line IV-IV in Fig. 2. Fig. 5 shows a cross-sectional view taken along line V -V in Fig. 2. In the drawings, the arrow x direction is defined as a width direction of the diaper, and the arrow y is defined as a front-back direction of the diaper. The direction perpendicular to the plane formed by the arrows x and y is defined as a thickness direction z.

A pants-type disposable diaper 1 comprises a pants-shaped outer member 2 having a front part P, a back part Q, and a crotch part R positioned between the front part P and the back part Q, and having a waist opening 3 and a pair of leg openings 4 formed by joining the front part P and the back part Q. The pants-shaped outer member 2 comprises an inner sheet 5 and an outer sheet 6, and the inner sheet 5 and the outer sheet 6 are made of a spunbonded nonwoven fabric produced by melting a polymer composition containing polyolefin and/or polyester and a nonionic surfactant.

The pants-type disposable diaper 1 comprises an absorbent main body 8 disposed on an inner surface of the pants-shaped outer member 2 at the crotch part R and comprising a top sheet 9, a back sheet 10, and an absorbent core 11 disposed between the top sheet 9 and the back sheet 10 (Fig. 3). The top sheet 9 is placed so as to face a wearer's skin, and allows excrement such as urine and the like to permeate through. The excrement that permeated the top sheet 9 is accommodated in the absorbent core 11. The back sheet 10 is attached to the inner sheet 5 of the pants-shaped outer member 2, and prevents the excrement from leaking outside.

Rising flaps 12 are provided along an edge of opposite sides, with respect to the width direction x, of the absorbent main body 8. The rising flap 12, which extends in the front-back direction y of the diaper, is joined astride the top sheet 9 and the back sheet 10. A rising elastic member 13 is disposed at an inner end in the width direction x of the rising flap 12. A rising gather which rises upward (toward a wearer) is formed from the rising flap 12 due to a shrinkage force of the rising elastic member 13, thereby preventing lateral leakage of urine and the like. An inner surface of the rising flaps 12 is joined to the top sheet 9 at front and back ends of the absorbent main body 8, thereby preventing leakage of urine and the like outward in the front-back direction y.

A plurality of body elastic members 14 are disposed between the inner sheet 5 and the outer sheet 6 at the front part P and the back part Q of the pants-shaped outer member 2 so as to extend in the width direction x of the diaper (Figs. 1,2 and 4). The body elastic member 14 is adhered to the inner sheet 5 and the outer sheet 6 with a hot-melt adhesive 15 applied around the body elastic member 14. The inner sheet 5 and the outer sheet 6 are not adhered each other at a position between the adjacent body elastic members 14, as shown in Fig. 5.

Concerning application of the hot-melt adhesive 15 around the body elastic member 14, the hot-melt adhesive 15 may be applied to the entire circumference of the body elastic member 14 as shown in Fig. 6A, or the hot-melt adhesive 15 may be applied to substantially 3/4 or half of the circumference of the body elastic member 14 as shown in Figs. 6B or 6C. In light of reliable adhesion of the body elastic member 14 to the inner sheet 5 and the outer sheet 6, the hot-melt adhesive 15 is preferably applied to at least half or more of the circumference of the body elastic member 14. Application of the hot-melt adhesive to the entirety or a part of the circumference of the elastic member as shown in Figs. 6A to 6C is not limited to application to the body elastic member 14.

In Figs. 2 and 4, an end-holding sheet 7 is provided at the front part P and the back part Q of the pants-type disposable diaper 1 so as to cover a front or back end of the absorbent main body 8. The end-holding sheet 7 is provided so that a wearer is less likely to feel discomfort that is caused by the front or back end of the absorbent main body 8 coming into direct contact with a wearer's skin. In Figs. 4 and 5, the end-holding sheet 7 is adhered to the front or back end of the absorbent main body 8 and the inner sheet 5 with a hot-melt adhesive 16 so as to cover the front or back end of the absorbent main body 8 and the entirety of the body elastic members 14.

A plurality of waist elastic members 18 are disposed to the pants-shaped outer member 2 so as to extend along a waist opening edge 17, that is an edge of the waist opening 3, in the width direction x of the diaper, as shown in Figs. 1, 2 and 4. The outer sheet 6 is folded back toward the inner sheet 5 at the waist opening edge 17 of the pants-shaped outer member 2, and the folded outer sheet 6 is adhered to the end-holding sheet 7 with a hot-melt adhesive 20, as shown in Fig. 5. The waist elastic members 18 are interposed between the folded and unfolded parts of the outer sheet 6, and adhered to the outer sheet 6 with the hot-melt adhesive 19 applied around the waist elastic member 18. In order to reliably join the waist elastic members 18 to the outer sheet 6, preferably, a hot-melt adhesive 21 is applied to the folded part of the outer sheet 6 and the waist elastic members 18 are adhered to the outer sheet 6 with the hot-melt adhesive 21.

Leg elastic members 23 and 24 are disposed between the inner sheet 5 and the outer sheet 6 along the leg opening edges 22, that is edges of the leg openings 4, of the pants-shaped outer member 2 , as shown in Fig. 2. The leg elastic member consists of a front leg elastic member 23 provided so as to extend across the crotch part R and along the leg opening edges on a front side of the diaper; and a back leg elastic member 24 provided so as to extend across the crotch part R and along the leg opening edges on a back side of the diaper. By the front leg elastic member 23 and the back leg elastic member 24, the leg elastic member is provided along substantially the entire circumference of the leg opening edges 22.

### REFERENCE SIGNS LIST

- 1:: a pants-type disposable diaper
- 2:: a pants-shaped outer member
- 5:: an inner sheet
- 6:: an outer sheet
- 8:: an absorbent main body
- 9:: a top sheet
- 10:: a back sheet
- 11:: an absorbent core
- 14:: a body elastic member
- 15:: a hot-melt adhesive
- 18:: a waist elastic member
- 23, 24:: a leg elastic member

## Claims

1. A pants-type disposable diaper (1) comprising:
a pants-shaped outer member (2) having a front part (P), a back part (Q), and a crotch part (R) positioned between the front part (P) and the back part (Q), and having a waist opening (3) and a pair of leg openings (4) formed by joining the front part (P) and the back part (Q); and
an absorbent main body (8) disposed on an inner surface of the pants-shaped outer member (2) at the crotch part (R), and comprising a top sheet (9), a back sheet (10), and an absorbent core (11) disposed between the top sheet (9) and the back sheet (10); wherein
the pants-shaped outer member (2) comprises an inner sheet (5) and an outer sheet (6), and
the inner sheet (5) and the outer sheet (6) are made of a spunbonded nonwoven fabric produced by melting a polymer composition containing polyolefin and/or polyester and a nonionic surfactant.

2. The pants-type disposable diaper (1) according to claim 1,
wherein the outer sheet (6) is folded back toward the inner sheet (5) at an edge (17) of the waist opening (3) of the pants-shaped outer member (2).

3. The pants-type disposable diaper (1) according to claim 1 or 2, further comprising:
a plurality of body elastic members (14) disposed between the inner sheet (5) and the outer sheet (6) at the front part (P) and the back part (Q) of the pants-shaped outer member (2) so as to extend in a width direction of the diaper, wherein
the body elastic members (14) are adhered to the inner sheet (5) and/or the outer sheet (6), and the inner sheet (5) and the outer sheet (6) are not adhered each other at a position between the adjacent body elastic members (14).

4. The pants-type disposable diaper (1) according to any one of claims 1 to 3,
wherein the nonwoven fabrics of the inner sheet (5) and the outer sheet (6) have a fineness of 1.0 dtex or more and less than 1.5 dtex.

## Patentansprüche

1. Wegwerfwindel vom Höschentyp (1) umfassend:
einen höschenförmigen Außenbestandteil (2), der ein Vorderteil (P), ein Rückenteil (Q) und ein Schrittteil (R), das zwischen dem. Vorderteil (P) und dem Rückenteil (Q) positioniert ist, aufweist und eine Taillenöffnung (3) und ein Paar Beinöffnungen (4) aufweist, die durch Zusammenfügen des Vorderteils (P) und des Rückenteils (Q) gebildet werden; und
einen absorptionsfähigen Hauptkörper (8), der sich auf einer Innenfläche des höschenförmigen Außenbestandteils (2) am Schrittteil (R) befindet und eine Deckschicht (9), eine Rückenschicht (10) und einen absorptionsfähigen Kern (11) umfasst, der sich zwischen der Deckschicht (9) und der Rückenschicht (10) befindet; wobei
der höschenförmige Außenbestandteil (2) eine Innenschicht (5) und eine Außenschicht (6) umfasst und
die Innenschicht (5) und die Außenschicht (6) aus Spinnvliesstoff bestehen, der durch Schmelzen einer Polymerzusammensetzung, die Polyolefin und/oder Polyester und ein nichtionisches Tensid enthält, hergestellt ist.

2. Wegwerfwindel vom Höschentyp (1) nach Anspruch 1, wobei die Außenschicht (6) auf die Innenschicht (5) an einer Kante (17) der Taillenöffnung (3) des höschenförmigen Außenbestandteils (2) zurückgeschlagen ist.

3. Wegwerfwindel vom Höschentyp (1) nach Anspruch 1 oder 2, des Weiteren umfassend:
mehrere körperelastische Bestandteile (14), die sich zwischen der Innenschicht (5) und der Außenschicht (6) am Vorderteil (P) und Rückenteil (Q) des höschenförmigen Außenbestandteils (2) so befinden, dass sie sich in einer Breitenrichtung der Windel erstrecken, wobei
die körperelastischen Bestandteile (14) an der Innenschicht (5) und/oder die Außenschicht (6) haften und die Innenschicht (5) und die Außenschicht (6) an einer Stelle zwischen den aneinanderliegenden, körperelastischen Bestandteile (14) nicht aneinander haften.

4. Wegwerfwindel vom Höschentyp (1) nach einem der Ansprüche 1 bis 3,
wobei die Vliesstoffe der Innenschicht (5) und der Außenschicht (6) eine Feinheit von 1,0 dtex oder mehr und weniger als 1,5 dtex aufweisen.

## Revendications

1. Couche jetable de type culotte (1) comprenant :
un élément externe en forme de culotte (2) ayant une partie avant (P), une partie arrière (Q), et une partie entrejambe (R) positionnée entre la partie avant (P) et la partie arrière (Q) et ayant une ouverture de taille (3) et une paire d'ouvertures de jambe (4) formées par la liaison de la partie avant (P) et de la partie arrière (Q) ; et
un corpus principal absorbant (8) disposé sur une surface interne de l'élément externe en forme de culotte (2) au niveau de la partie entrejambe (R), et comprenant une feuille supérieure (9), une feuille arrière (10), et une partie centrale absorbante (11) disposée entre la feuille supérieure (9) et la feuille arrière (10) ; dans laquelle
l'élément externe en forme de culotte (2) comprend une feuille interne (5) et une feuille externe (6), et
la feuille interne (5) et la feuille externe (6) sont réalisées en un tissu non tissé par filage direct produit par fusion d'une composition polymère contenant une polyoléfine et/ou un polyester et un surfactant non ionique.

2. Couche jetable de type culotte (1) selon la revendication 1, dans laquelle la feuille externe (6) est repliée vers la feuille interne (5) au niveau d'un bord (17) de l'ouverture de taille (3) de l'élément externe en forme de culotte (2).

3. Couche jetable de type culotte (1) selon la revendication 1 ou 2, comprenant en outre :
une pluralité d'éléments élastiques de corps (14) disposés entre la feuille interne (5) et la feuille externe (6) au niveau de la partie avant (P) et de la partie arrière (Q) de l'élément externe en forme de culotte (2) de manière à ce qu'ils s'étendent dans une direction de largueur de la couche, dans laquelle
les éléments élastiques de corps (14) sont collés à la feuille interne (5) et/ou à la feuille externe (6), et la feuille interne (5) et la feuille externe (6) ne sont pas collées l'une à l'autre au niveau d'une position entre les éléments élastiques de corps (14) adjacents.

4. Couche jetable de type culotte (1) selon l'une quelconque des revendications 1 à 3,
dans laquelle les tissus non tissés de la feuille interne (5) et de la feuille externe (6) ont une finesse d'au moins 1,0 dtex et inférieur à 1,5 dtex.
